# EUROPÄISCHE PATENTSCHRIFT

(11) **EP 2 103 293 B1**
(45) Veröffentlichungstag und Bekanntmachung des Hinweises auf die Patenterteilung: **09.05.2018**
(21) Anmeldenummer: 09003754.0
(22) Anmeldetag: 16.03.2009
(51) Int. Cl.: A61F 13/58, A61F 13/62

(54) **Windelverschlussband**
Fastener for diaper
Bande de fermeture pour couche

(30) Priorität: 16.03.2008 DE 102008014223
(43) Veröffentlichungstag der Anmeldung: 23.09.2009
(73) Patentinhaber: Lohmann-koester GmbH & Co. KG, 96146 Altendorf (DE)
(72) Erfinder: Neugebauer, Robert, 91077 Neunkirchen (DE); Drozdziok-Weinhardt, Magdalena, 91056 Erlangen (DE); Schober,Uwe, 96179 Rattelsdorf (DE)
(74) Vertreter: Reuther, Martin

(56) Entgegenhaltungen:
- EP-A2- 0 951 888
- WO-A1-97/25954
- US-A1- 2003 032 359

## Beschreibung

Die Erfindung betrifft ein Windelverschlussband mit einem Permanentbereich und mit einem Fasteningbereich, wobei der Fasteningbereich einen mechanischen Verschlussbereich umfasst.

So offenbaren beispielsweise die EP 0 813 851 A1 und die WO°00/13641 A1 Windelverschlussbänder, bei welchen mechanische Verschlussbereiche oder klebende Verschlussbereiche dazu dienen, in einem Befestigungsbereich eine Windel öffenbar zu verschließen. Je nach konkreter Ausgestaltung können hierbei Landeelemente vorgesehen sein, die auf einer entsprechenden Oberfläche einer Windel beim ersten Verschließen abgelegt werden, um so einen ausreichend stabilen Verschluss zu gewährleisten. Auch die WO 2004/105671 A1 offenbart ein derartiges Landeelement in Verbindung mit einem klebenden Verschlussbereich, wobei jedoch zwischen dem klebenden Verschlussbereich und dem Permanentbereich ein elastischer Bereich vorgesehen ist, um einen verbesserten Tragekomfort und eine bessere Anpassungsmöglichkeit zu gewährleisten. Weiteren Stand der Technik offenbart die US 2003/0032359 A1. WO 97/25954 offenbart ein Windelverschlussband mit einem Permanentbereich und mit einem Fasteningbereich, wobei der Fasteningbereich einen mechanischen Verschlussbereich umfasst,dadurch gekennzeichnet, dass der mechanische Verschlussbereich zwischen einem permanentbereichseitig des mechanischen Verschlussbereiches angeordneten elastischen Bereich und einem auf einer dem Permanentbereich abgewandten Seite des mechanischen Verschlussbereiches angeordneten klebenden Verschlussbereich, der in dem Fasteningbereich liegt, angeordnet ist.

Die Kombination sowohl klebender als auch mechanischer Verschlussbereiche in Verbindung mit einem elastischen Bereich offenbart die WO 2005/074852 A1. Hierbei sind klebende und mechanische Verschlussbereiche in einem gleichen Bereich des Windelverschlussbandes vorgesehen.

Es ist Aufgabe vorliegender Erfindung, ein gattungsgemäßes Windelverschlussband bereitzustellen, welches gegenüber dem Stand der Technik einen sichereren Verschluss aufweist bzw. welches auch mit kleineren Verschlussbereichen dem Stand der Technik entsprechend sichere Verschlussmöglichkeiten bietet.

Als Lösung wird ein Windelverschlussband mit den Merkmalen des Anspruchs 1 vorgeschlagen.

Hierbei ermöglicht die Anordnung des mechanischen Verschlussbereichs zwischen dem permanentbereichseitig angeordneten elastischen Bereich und dem befestigungsbereichseitig angeordneten klebenden Verschlussbereich, dass durch den elastischen Bereich, welcher ohne Weiteres beim Schließen eines entsprechenden Verschlusses von einem Nutzer unter Spannung gesetzt werden kann, der mechanische Verschlussbereich unter einer entsprechenden Zugspannung gehalten wird, so dass entsprechenden Scherkräfte den mechanischen Verschluss permanentbereichsseitig verhältnismäßig fester verankern, während der klebende Verschlussbereich den mechanischen Verschluss befestigungsbereichseitig niederhaltend wirkt. Insbesondere können hierdurch Peel-Off-Effekte, welche durch die in das Windelverschlussband von dem unter Zugspannung stehenden elastischen Bereich in den mechanischen Verschlussbereich eingetragen werden und welche insbesondere auf der dem Befestigungsbereich abgewandten Seite zu einem Heben des mechanischen Verschlusses führen und hierdurch dessen Verschlusskraft mindern, vermieden werden, so dass das gesamte Windelverschlussband besser schließt.

In Abweichung insbesondere von der WO 2005/074852 A1 kann der klebende Verschlussbereich insbesondere auf der dem elastischen Bereich abgewandten Seite des mechanischen Verschlussbereichs, der naturgemäß am ehesten dazu neigt auf Grund der inneren Spannungen beim Tragen der Windel abzuheben, besonders wirksam seine niederhaltende Funktion ausüben und somit gerade dort ein Abheben verhindern. Die Lösung nach der WO 2005/074852 A1 kann hier gerade nur bedingt weiter helfen, da der klebende Bereich durch die mechanischen Verschlusselemente, die in einer gewissen Höhe von dem Windelverschlussband abstehen, nur erschwert mit einer Windel bzw. mit einem Landebereich des Befestigungsbereichs auf der Windel in Kontakt kommen kann.

Eine bevorzugte Ausführungsvariante sieht vor, dass zwischen den beiden Verschlussbereichen und dem Permanentbereich ein elastisches Element, welches den elastischen Bereich des Windelverschlussbandes bildet, angeordnet ist. Mit einem zusätzlichen elastischen Element kann der elastische Bereich an dem Windelverschlussband konstruktiv besonders einfach bereitgestellt werden. Das elastische Element kann beispielsweise als Folienmaterial oder als massiver elastischer Streifen vorgesehen sein.

Um die Verschlussfähigkeit des Windelverschlussbandes besonders positiv zu beeinflussen, ist es vorteilhaft, wenn der elastische Bereich, ein elastisches Element umfasst, welches auf der Seite, auf welcher die Verschlusselemente vorgesehen sind, hervorkragt. Hierbei beschreibt der Begriff "hervorkragen" eine Anordnung, bei welcher das elastische Element an der Seite der Verschlusselemente über ein Trägerband des Windelverschlussbandes hervorstehen und vorzugsweise entsprechend auf dieser Seite des Trägerbands an diesem befestigt bzw. mit diesem verbunden ist. Andererseits ist es auch denkbar, dass das elastische Element einstückig in dem Trägerband ausgebildet ist und Fasteningbereich und Permanentbereich ggf. stabilisiert sind, was beispielsweise durch geeignete Baugruppen oder durch eine nicht erfolgte Aktivierung von elastischen Komponenten in diesen Bereichen realisiert werden kann.

Umfasst der elastische Bereich kumulativ oder alternativ ein elastisches Element, welches stärker ausgebildet ist als ein Trägerband des Windelverschlussbandes, kann hierdurch insbesondere ein gleichförmigerer Gesamtaufbau erzielt werden, da in der Regel die Verschlusselemente, insbesondere etwaige Hakenmaterialien bzw. zusätzliche Target- oder Releasebänder zu verhältnismäßig großen Dicken führen. Ein derartig vergleichmäßigter Aufbau erleichtert insbesondere ein Aufrollen von Bandmaterial, wenn dieses als Halbzeug gelagert und versandt werden soll, bevor dann die entsprechenden Bandabschnitte zu Windelverschlussbändern abgeschnitten und an einer Windel appliziert werden sollen.

Es versteht sich, dass der elastische Bereich beziehungsweise das elastische Element aus einer Vielzahl von Materialien hergestellt sein kann, welche geeignete elastische Fähigkeiten besitzen. Um unabhängig hiervon immer eine besonders gute Haptik auch in dem Bereich des elastischen Elementes leisten zu können, ist es vorteilhaft, wenn der elastische Bereich ein elastisches Element umfasst, welches kaschiert ist. In vorliegendem Zusammenhang bezeichnet der Begriff "kaschieren" jedwedes großflächiges Überdecken einer Materiallage mit einer anderen Materiallage, welche dann entsprechend als Kaschierung bezeichnet wird. Hierbei kann die Kaschierung über eine Klebeverbindung oder über sonstige Verbindungsprozesse mit der kaschierten Materiallage verbunden sein. Auch kann beispielsweise die Materiallage selbst auf die Kaschierung, beispielsweise mittels eines Extrusionsprozesses, aufgebracht worden sein. So ist es beispielsweise denkbar, dass ein elastischer Film, beispielsweise ein erwärmter thermoplastisches Elastomer, auf eine Vlies- oder Nonwovenlage in verflüssigter oder in fließfähiger Form aufgebracht und auf diese Weise ein mit Vlies oder Nonwoven kaschierter elastischer Film bereitgestellt wird, wobei das Vlies bzw. Nonwoven eigenstabil aber auch nicht eigenstabil und durch den Film stabilisiert sein kann.

Eine weitere bevorzugte Ausführungsvariante sieht vor, dass der elastische Bereich ein elastisches Element umfasst, welches an ein Trägerband des Windelverschlussbandes anextrudiert ist. Hierdurch kann das elastische Element konstruktiv einfach auf vielfältige Weise an dem Windelverschlussband angebracht werden.

Umfasst der elastische Bereich ein elastisches Element, welches einen elastisch verformbaren Kernbereich aufweist, welcher in Längserstreckung des Windelverschlussbandes also quer zur Maschinenrichtung wenigstens 2,5 mm, vorzugsweise 3,0 mm, misst, kann betriebssicher eine elastisch wirksame Mindestbreite an dem elastischen Element bereitgestellt werden. Ist der elastisch verformbare Kernbereich schmaler ausgebildet, besteht die Gefahr, dass der elastische Bereich zu wenig elastische Substanz aufweist, um die gewünschten elastischen Eigenschaften an dem Windelverschlussband betriebssicher bereitzustellen.

Der mechanische Verschlussbereich und der klebende Verschlussbereich können mittels des elastischen Elementes gemeinsam an dem Permanentbereich befestigt sein.

Insgesamt können mit dem elastischen Bereich beziehungsweise mit dem elastischen Element funktionale Verbesserungen an dem Windelverschlussband erzielt werden, selbst dann, wenn beispielsweise weniger hochwertige Backsheet-Qualitäten an einer Windel verwendet werden, da der elastische Bereich in das Windelverschlussband eingeleitete Kraftspitzen besonders für gut aufnehmen bzw. kompensieren kann. Insofern kann die Gefahr verringert werden, dass es an dem Backsheet der Windel zu unerwünschten Faserausrissen kommen beziehungsweise ein pop-off-Effekt bei einer Kraftentlastung entstehen kann. Darüber hinaus kann eine Verbesserung des Tragekomforts einer Windel durch die verlängerte Reichweite des Windelverschlussbandes erzielt werden, insbesondere im Hinblick auf eine vergrößerte Auflagefläche. Besonders vorteilhaft ist es, dass das Windelverschlussband mit Vorspannung auf eine Windel aufgebracht werden kann.

Insbesondere kann ggf. durch eine Kombination der drei Funktionseigenschaften "klebend, mechanisch, elastisch" an dem Windelverschlussband die Größe von Funktionsflächen hinsichtlich der klebenden und mechanischen Verschlussbereiche reduziert werden, so dass das Windelverschlussband wesentlich kompakter und damit auch kostengünstiger bereitgestellt werden kann.

Es versteht sich, dass es eine Vielzahl an Ausführungsformen bezüglich des Windelverschlussbandes geben kann. Beispielsweise wird das elastische Element mit einem Vlies abgedeckt bzw. kaschiert, wie vorstehend bereits erläutert. Das Windelverschlussband kann auch releasebandfrei erstellt werden. Ebenso kann etwa ein Klett des mechanischen Verschlussbereiches in Wellenform oder ähnliches ausgeführt sein.

An dieser Stelle sei noch erwähnt, dass es vorteilhaft ist, wenn das Windelverschlussband ein Trägerband mit einem fasteningbereichseitigen Trägerbandteil und einem permanentbereichseitigen Trägerbandteil aufweist, wobei der fasteningbereichseitige Trägerbandteil und der permanentbereichseitige Trägerbandteil mehr als 2,00 mm, vorzugsweise mehr als 3,00 mm, voneinander beabstandet angeordnet sind. Hierdurch bleibt zwischen den beiden Trägerbandteilen genügend Bauraum vorhanden, um das elastische Element beziehungsweise den elastischen Bereich am Windelverschlussband zu realisieren.

Darüber hinaus kann speziell der Fasteningbereich des Windelverschlussbandes, insbesondere hinsichtlich eines 3-lagig ausgebildeten Funktionstapes, besonders vorteilhaft ausgebildet werden, wenn der Fasteningbereich einen klebenden Fasteningbereichsaufbau mit einer Höhe HK und einen mechanischen Fasteningbereichsaufbau mit einer Höhe HM aufweist, wobei HM ≤ 2,0 x HK, vorzugsweise HM ≤ 1,7 x HK, ist. Hierdurch kann sichergestellt werden, dass der mechanische Fasteningbereichsaufbau gegenüber dem klebenden Fasteningbereichsaufbau nicht zu hoch ausgebildet wird, so dass der klebende Verschlussbereich gut etwa an einem Frontsheet einer Windel kleben kann.

In diesem Zusammenhang umfasst der klebende Fasteningbereichsaufbau vorzugsweise diejenigen Komponenten des Windelverschlussbandes, welche an einer einem Trägerband abgewandten Seite eines Montageklebstoffes zumindest teilweise im klebenden Verschlussbereich angeordnet sind.

Der Begriff "Montageklebstoff" beschreibt vorliegend einen Kleber, der zum dauerhaften Verkleben einzelner Windelverschlussbandkomponenten vorgesehen ist. Dieser Montageklebstoff spielt hinsichtlich der klebenden Verschlussmöglichkeiten des Windelverschlussbandes jedoch keine wesentliche Rolle. Hierfür ist idealerweise ausschließlich der Fasteningklebstoff des klebenden Verschlussbereiches vorgesehen. Als Montageklebstoff kann beispielsweise ein Heißkleber zur Anwendung kommen, der sich in kaltem Zustand an seiner Oberfläche nicht mehr klebrig abfühlt und lediglich mit Komponenten verklebt bleibt, mit denen er in heißem Zustand in Kontakt gebracht wurde.

In einer konkreten Ausführungsform kann das Windelverschlussband vorteilhafter Weise derart aufgebaut sein, dass der klebende Fasteningbereichsaufbau im Wesentlichen einen Fasteningklebstoff, ein Targetelement, einen Targetelementklebstoff, ein Releaseband, und ein Releaseklebstoff umfasst. Durch einen solchen Aufbau wird ein besonders ausgeglichener Dickenaufbau des vorliegenden Windelverschlussbandes insbesondere bezüglich des Fasteningbereiches erzielt, wobei der mechanische Verschlussbereich in seiner Höhe den Aufbau des danebenliegenden klebenden Verschlussbereiches nicht zu stark überschreiten sollte, um betriebssicher gewährleisten zu können, dass sowohl der klebende als auch der mechanische Verschlussbereich optimale Verschlusseigenschaften entfalten kann.

Während das zuvor beschriebene Höhenverhältnis zwischen dem klebenden Fasteningbereichsaufbau und dem mechanischen Fasteningbereichsaufbau im Speziellen eher hinsichtlich eines Windelschlussbandes mit einem 3-lagigen Funktionstape mit einer Fasteningkomponentenlage, mit einer Targetkomponentenlage und mit einer Releasekomponentenlage vorteilhaft sein kann, können sich bei Windelverschlussbändern mit einem alternativ aufgebauten Funktionstape auch andere Aufbauverhältnisse vorteilhaft darstellen.

Alternativ kann es hinsichtlich eines Winkelverschlussbandes mit einem 2-lagigen Funktionstape somit günstig sein, wenn das 2-lagige Funktionstape mit einer Fasteningkomponentenlage und mit einer Releasekomponentenlage ausgestattet ist, wobei der Fasteningbereich einen klebenden Fasteningbereichsaufbau mit einer Höhe HK und einen mechanischen Fasteningbereichsaufbau mit einer Höhe von HM aufweist und wobei HM ≤ 3,5 x HK, vorzugsweise HM ≤ 3,0 x HK, ist.

Es versteht sich, dass der klebende Verschlussbereich und der mechanische Verschlussbereich in vielfältiger Weise weiter vorteilhaft gestaltet sein können. Insofern sieht eine weitere vorteilhafte Ausführungsvariante ein Windelverschlussband mit einem fasteningbereichseitigen Erstreckungsbereich vor, in welchem der klebende Verschlussbereich bis an den mechanischen Verschlussbereich heran reicht, wobei in dem Erstreckungsbereich ein Montageklebstoff angeordnet ist, mittels welchem vorzugsweise beide Verschlussbereiche des Windelverschlussbandes an einem Trägerband des Windelverschlussbandes befestigt sind. Der Begriff "Erstreckungsbereich" bezeichnet einen fasteningbereichseitigen Bereich an dem Windelverschlussband, in welchem der klebende und der mechanische Verschlussbereich zumindest teilweise angeordnet sein können. Im Speziellen kann sich der Erstreckungsbereich auch vollständig unterhalb des klebenden Verschlussbereiches und des mechanischen Verschlussbereiches vorgesehen sein. Vorzugsweise beginnt der Erstreckungsbereich an einem dem klebenden Verschlussbereich zugewandte Ende des mechanischen Verschlussbereiches und erstreckt sich bis weit unter den klebenden Verschlussbereich. Durch einen derartigen Erstreckungsbereich kann das Windelverschlussband wesentlich einfacher hergestellt werden, da bei der Herstellung des Windelverschlussbandes beziehungsweise beim Anbringen des klebenden Verschlussbereiches beziehungsweise des mechanischen Verschlussbereiches idealerweise nicht darauf geachtet werden muss, wie präzise die beiden Verschlussbereiche auf dem Montageklebstoff aufgebracht werden müssen. Hierdurch kann die Herstellung insbesondere des Fasteningbereichs des Windelverschlussbandes wesentlich vereinfacht werden.

Es versteht sich, dass es bei einem derart gestalteten Erstreckungsbereich möglich ist, dass der klebende Verschlussbereich und der mechanische Verschlussbereich unmittelbar aneinander anliegen können. Insofern können der mechanische Verschlussbereich und der klebende Verschlussbereich bündig nebeneinander auf dem Trägerband des Windelverschlussbandes angeordnet sein.

Eine demgegenüber andere Ausführungsvariante sieht jedoch vor, dass der mechanische Verschlussbereich und der klebende Verschlussbereich beabstandet voneinander auf dem Trägerband des Windelverschlussbandes angeordnet sind. Sind die beiden unterschiedlichen Verschlussbereiche beabstandet voneinander auf dem Trägerband angeordnet, können sie wesentlich unkomplizierter an dem Trägerband appliziert werden. Insofern kann sich ein Herstellungsverfahren entsprechend vereinfachen, so dass das neue Windelverschlussband auch auf herkömmlichen Produktionseinrichtungen hergestellt werden kann.

Eine ergänzende vorteilhafte Ausführungsvariante sieht vor, dass der mechanische Verschluss mechanische Verschlusselemente umfasst, die von einem Trägerband des Windelverschlussbandes mit einer Verschlusselementhöhe abstehen, und dass der klebende Verschlussbereich in einem dieser Verschlusselementhöhe entsprechenden Abstand und/oder einem größeren Abstand von dem mechanischen Verschlussbereich auf der dem Permanentbereich abgewandten Seite vorgesehen ist. Auch hierdurch kann gewährleistet werden, dass beide Verschlusse gleichermaßen mit einer entsprechenden Oberfläche ausreichend in Kontakt kommen können, um ihre Aufgabe in erfindungsgemäßer Weise besonders geeignet zu erfüllen. Beträgt der Abstand zwischen dem klebenden Verschlussbereich und dem mechanischen Verschlussbereich mindestens einen Wert, welcher der Verschlusselementhöhe entspricht, kann der klebende Verschlussbereich besonders großflächig und vorteilhaft beispielsweise an einem Frontsheet einer Windel aufgeklebt werden, obwohl sich neben dem klebenden Verschlussbereich mechanische Verschlusselemente befinden, die radial über den Fasteningklebstoff des klebenden Verschlussbereiches überstehen können. Insoweit kann ggf. im Bereich des Abstandes, wie bereits vorstehend angedeutet, ein Montageklebstoff vorgesehen ist, über welchen das mechanische Verschlusselement und das klebende Verschlusselement miteinander verbunden sind.

Darüber hinaus ist es vorteilhaft, wenn die mechanischen Verschlusselemente des mechanischen Verschlussbereiches auf wenigstens einem Trägerelement, mit welchem die mechanischen Verschlusselemente einstückig verbunden sind und welches eine Trägerelementhöhe aufweist, angeordnet sind, und dass die Verschlusselementhöhe zumindest die Trägerelementhöhe sowie die Höhe der mechanischen Verschlusselemente über dem Trägerelement beträgt. Hierdurch kann mit den bereits vorstehend erläuterten Vorteilen ein besonders gleichförmiger Aufbau gewährleistet werden.

Wenn die Verschlussbereiche mittels eines Montageklebstoffes auf einem Trägerband des Windelverschlussbandes dauerhaft aufgeklebt sind, gestaltet sich die Herstellung des Windelverschlussbandes besonders einfach.

Auch ist es vorteilhaft, wenn sich der Montageklebstoff auf einem Trägerband sowohl bis unter dem klebenden Verschlussbereich als auch bis unter den mechanischen Verschlussbereich erstreckt. Hierdurch sind beiden Verschlussbereiche außergewöhnlich betriebssicher mittels eines einzigen Montageklebstoffstreifens auf dem Trägerband befestigt.

In der Praxis hat es sich als vorteilhaft erwiesen, wenn der klebende Verschlussbereich neben dem mechanischen Verschlussbereich auf einem Trägerband des Windelverschlussbandes an einer dem elastischen Bereichs abgewandter Seite des mechanischen Verschlussbereiches angeordnet ist. Hierdurch kann eine besonders vorteilhafte pop-off-Sicherung an dem Windelverschlussband gewährleistet werden.

Außerordentlich hochwertige Verschlusseigenschaften können an dem Windelverschlussband mit einem Breitenverhältnis "mechanischer Verschlussbereich/klebender Verschlussbereich" von mindestens 1:8 und höchstens 4:1 erzielt werden. Durch ein solches Breitenverhältnis können der mechanische Verschlussbereich und der klebende Verschlussbereich bifunktional hervorragend beispielsweise mit einem Frontsheet einer Windel wechselwirken.

Hinsichtlich der Fasteningkomponentenlage und der Targetkomponentenlage ist ein Breitenverhältnis "mechanischer Verschlussbereich/Targetelement" von mindestens 1:9 und höchstens 2:1 vorteilhaft. Durch ein solches Breitenverhältnis kann ebenfalls betriebssicher gewährleistet werden, dass der mechanische Verschlussbereich und der klebende Verschlussbereich bifunktional sehr gut beispielsweise mit einem Frontsheet einer Windel wechselwirken können.

Und kumulativ oder alternativ ist es vorteilhaft, wenn der mechanische Verschlussbereich auch klebende Verschlussbereiche aufweist. Sind derartige klebende Verschlussbereiche beispielsweise innerhalb des mechanischen Verschlussbereiches integriert, können die Verschlusseigenschaften des Windelverschlussbandes zusätzlich variiert beziehungsweise zusätzlich erhöht werden. Insbesondere könnte bei gleichbleibender Verschlussqualität des Windelverschlussbandes der klebende Verschlussbereich außerhalb des mechanischen Verschlussbereiches zumindest kleiner gestaltet werden, wodurch das Windelverschlussband insgesamt noch kompakter gebaut werden kann.

Des Weiteren können innerhalb des Windelverschlussbandes auftretende Kraftspitzen zusätzlich kompensiert werden, wenn der mechanische Verschlussbereich ebenfalls wenigstens teilweise elastisch ausgebildet ist.

Vorteilhaft ist es, wenn der klebende Verschlussbereich mit einem übertragbaren Targetelement zusammenwirkt. Hierdurch kann insbesondere ein Windelverschlussband mit einem 3-lagigen Funktionstape hinsichtlich eines Halbzeugs konstruktiv einfach bereitgestellt werden.

Bei dem Windelverschlussband kann die Applikation an einer Windel vereinfacht und dessen stabile Befestigung einfach sichergestellt werden, wenn das Windelverschlussband ein Releaseband aufweist, welches das mechanische Verschlusselement überdecken kann.

Weist das Windelverschlussband alternativ bzw. kumulativ hierzu ein Releaseband auf, welches das elastische Element überdeckt, kann hierdurch ein stabiler Aufbau des Windelverschlussbandes, insbesondere auch vor seiner Applikation an einer Windel, gewährleistet werden.

Weitere Vorteile, Ziele und Eigenschaften vorliegender Erfindung werden anhand nachfolgender Beschreibung anliegender Zeichnung erläutert, in welcher beispielhaft zwei Windelverschlussbänder dargestellt sind, bei welchen jeweils ein mechanischer Verschlussbereich zwischen einem permanentbereichseitig des mechanischen Verschlussbereiches angeordneten elastischen Bereich und einem auf einer dem Permanentbereich abgewandten Seite des mechanischen Verschlussbereiches angeordneten klebenden Verschlussbereich angeordnet ist. Komponenten, welche in den Figuren wenigsten im Wesentlichen hinsichtlich ihrer Funktion übereinstimmen, können hierbei mit gleichen Bezugsziffern gekennzeichnet sein, wobei diese Komponenten nicht in allen Figuren beziffert und erläutert sein müssen. In der Zeichnung zeigen:
- Figur 1: ein erstes Windelverschlussband in schematischem Schnitt; und
- Figur 2: ein zweites Windelverschlussband in schematischem Schnitt.

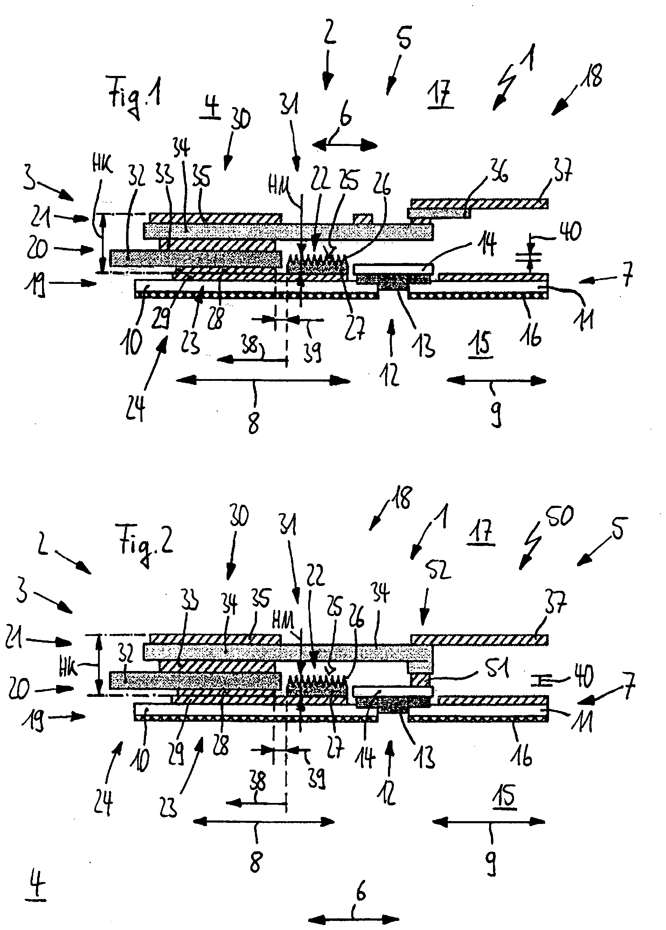

Das in der Figur 1 gezeigte Windelverschlussband 1 ist ein erstes Halbzeugstreifennutzen 2 von zwei Halbzeugstreifennutzen einer Halbzeugbahn 3 (hier nur eine Hälfte gezeigt), welcher sich in die Papierebene 4 (Maschinenrichtung) hinein erstreckt. Das aus der Halbzeugbahn 3 quasi als ein Streifen 5 entsprechend senkrecht zur Maschinenrichtung (Querrichtung) abgetrennte Windelverschlussband 1 erstreckt sich mit seiner Längserstreckung 6 in und entlang dieser Papierebene 4. Es versteht sich, dass in einer alternativen Ausführungsform die Halbzeigbahn auch derart ausgebildet sein, dass lediglich ein Halbzeugstreifennutzen durch ein Trennen senkrecht zur Maschinenrichtung bereitgestellt werden kann.

Das Windelverschlussband 1 weist ein Trägerband 7 auf, wobei das Trägerband 7 idealerweise als Film oder NonWoven ausgebildet ist. Ebenso kann es mehrlagig oder einlagig gewählt sein. Insbesondere kann auch ein eigenstabiles NonWoven oder ein durch einen Film stabilisiertes NonWoven oder eine mit einer textilen Haptik, beispielsweise durch Beflocken, versehene eigenstabile Schicht zur Anwendung kommen.

Das Windelverschlussband 1 kann hinsichtlich seiner Längserstreckung in Querrichtung 6 im Wesentlichen in einen Fasteningbereich 8 und in einen Permanentbereich 9 unterteilt werden. Hierbei findet sich in der Fasteningbereich 8 an einem ersten Trägerbandteil 10 und der Permanentenbereich 9 an einem zweiten Trägerbandteil 11 wieder. Zwischen dem ersten Trägerbandteil 10 und dem zweiten Trägerbandteil 11 ist ein elastischer Bereich 12 des Windelverschlussbandes 1 vorgesehen, der mittels eines an dem Trägerband 7 anextrudierten elastischen Element 13 konstruiert ist. An seiner dem Trägerband 7 abgewandten Seite weist das elastische Element 13 noch eine Kaschierung 14 auf. Es versteht sich, dass als elastisches Element in alternativen Ausführungsformen auch andere elastische Komponenten zur Anwendung kommen können.

Während das Trägerband 7 an einer ersten Seite 15 des Windelverschlussbandes 1 lediglich eine Filmschicht 16 aufweist, welche sich sowohl an dem ersten Trägerbandteil 10 als auch an dem zweiten Trägerbandteil 11 erstreckt, befinden sich an einer zweiten Seite 17 des Windelverschlussbandes 1 weitere wesentliche Komponenten des Windelverschlussbandes 1.

Im Allgemeinen kann festgehalten werden, dass es sich bei diesem Ausführungsbeispiel des Windelverschlussbandes 1 um ein 3-lagiges Funktionstape 18 handelt, welches eine Fasteningkomponentenlage 19, eine Targetkomponentenlage 20 und eine Releasekomponentenlage 21 aufweist. Hierbei liegen die Fasteningkomponentenlage 19, die Targetkomponentenlage 20 und die Releasekomponentenlage 21 gemäß der Darstellung nach der Figur 1 in einer senkrecht zur Maschinenrichtung und senkrecht zur Querrichtung weisenden Richtung aufeinander.

Im Fasteningbereich 8 sind sowohl ein mechanischer Verschlussbereich 22 als auch ein klebender Verschlussbereich 23 vorhanden, wodurch ein Verschließen des Windelverschlussbandes 1 besonders betriebssicher vorgenommen werden kann. Während der mechanische Verschlussbereich 22 eine hervorragende Sheer-off-Sicherung an dem Windelverschlussband 1 realisiert, kann mittels des klebenden Verschlussbereiches 23 hervorragend eine Pop-off-Sicherung am Windelverschlussband 1 erzielt werden.

Besonders vorteilhaft ist es, dass der mechanische Verschlussbereich 22 zwischen einem permanentbereichseitig des mechanischen Verschlussbereiches 22 angeordneten elastischen Bereich 12 und dem auf der dem Permanentbereich 9 abgewandten Seite 24 des mechanischen Verschlussbereiches 22 angeordneten klebenden Verschlussbereich 23 angeordnet ist. Derart weit außen angeordnet, kann der klebende Verschlussbereich 23 besonders gut ein Abheben der Fasteningkomponentenlage 19 verhindern, wenn mittels des Windelverschlussbandes 1 eine Windel ordnungsgemäß verschlossen ist.

Der mechanische Verschlussbereich 22 besteht bei diesem Ausführungsbeispiel aus einem mechanischen Verschlusselement 25, welches wiederum aus Haken 26 auf einem Hakenträgerelement 27 besteht. Der klebende Verschlussbereich 23 hingegen ist bei diesem Ausführungsbeispiel lediglich durch einen Fasteningkleber 28 realisiert.

Sowohl der Fasteningkleber 28 als auch das mechanische Verschlusselement 25 sind mittels eines Montageklebers 29 auf dem Trägerband 7 aufgeklebt. Insofern umfasst der Fasteningbereich 8 einen klebenden Fasteningbereichsaufbau 30 und einen mechanischen Fasteningbereichsaufbau 31, wobei der klebende Fasteningbereichsaufbau 30 eine Höhe HK und der mechanische Fasteningbereichsaufbau eine Höhe HM aufweist. Bei diesem Ausführungsbeispiel stehen der klebende Fasteningbereichsaufbau 30 und der mechanische Fasteningbereichsaufbau 31 in einem Verhältnis HM ≤ 1,7 x HK zueinander.

Der klebende Fasteningbereichsaufbau 30 umfasst hierbei diejenigen Komponenten des Windelverschlussverbandes 1, welche zumindest teilweise im klebenden Verschlussbereich 23 oberhalb des Montageklebers 29 angeordnet sind. Im speziellen umfasst der klebende Fasteningbereichsaufbau 30 den Fasteningkleber 28, ein Targetelement 32, einen Targetelementklebstoff 33, ein Releaseband 34 und einen Releasebandklebstoff 35. Hierbei gehört das Targetelement 32 und der Targetelementklebstoff 33 zu der Targetkomponentenlage 20 des Windelverschlussbandes 1 und das Releaseband 34 und der Releasebandklebstoff 35 zu der Releasekomponentenlage 21 des Windelverschlussbandes 1, wobei permanentbereichseitig 9 der Releasekomponentenlage 21 noch einen Zwischenträger 36 und eine zusätzliche Releaseklebstoffschicht 37 zuzuordnen ist.

Wie vorstehend bereits erläutert, sind der mechanische Verschlussbereich 22 und der klebende Verschlussbereich 23 mittels des Montageklebers 29 an dem Trägerband 7 des Windelverschlussbandes 1 unlösbar angeklebt. Hierbei zeichnet sich das vorliegende Windelverschlussband 1 durch einen fasteningbereichseitigen Erstreckungsbereich 38 aus, in welchem der klebende Verschlussbereich 23 bis an den mechanischen Verschlussbereich 22 heran reicht, wobei in dem Erstreckungsbereich 38 der Montagekleber 29 angeordnet ist, mittels welchem vorzugsweise beide Verschlussbereiche 22, 23 an dem Trägerband 7 des Windelverschlussbandes 1 angeklebt sind. Der Erstreckungsbereich 38 beginnt hierbei direkt an dem mechanischen Verschlusselement 25 und ist auf den klebenden Verschlussbereich 23 hin ausgerichtet.

In diesem Erstreckungsbereich 38 kann insbesondere der Fasteningkleber 28 beliebig nahe an den mechanischen Verschlussbereich 22 heran reichen beziehungsweise nahezu beliebig weit von dem mechanischen Verschlussbereich 22 beabstandet sein. Hierdurch gelingt es, während der Herstellung des Windelverschlussbandes 1 sowohl den mechanischen Verschlussbereich 22 als auch den klebenden Verschlussbereich 23 unkompliziert an das Trägerband 7 anzukleben, ohne darauf besonders achten zu müssen, dass die beiden Verschlussbereiche 22 beziehungsweise 23 präzise auf dem Montagekleber 29 appliziert werden müssen. Hierdurch vereinfacht sich die Herstellung des Windelverschlussbandes 1 erheblich.

Nach der Darstellung des Ausführungsbeispiels gemäß der Figur 1 ist ein Abstand 39 zwischen dem klebenden Verschlussbereich 23 und dem mechanischen Verschlussbereich 22 derart groß gewählt, dass der klebende Verschlussbereich 23 weiter entfernt von dem mechanischen Verschlussbereich 22 angeordnet ist als eine Verschlusselementhöhe 40 des mechanischen Verschlusselementes 25. Hierdurch kann gewährleistet werden, dass idealerweise der gesamte klebenden Verschlussbereich 23 vollflächig beispielsweise auf einem Zielgebiet an einem Frontsheet einer hier nicht gezeigten Windel ankleben kann.

Bei dem in der Figur 2 gezeigten weiteren Ausführungsbeispiel 50 weist ein diesbezüglich gezeigtes alternatives Windelverschlussband 1 einen ähnlichen Aufbau auf, wie das zuvor beschriebene Windelverschlussband 1 aus der Figur 1. Die beiden Windelverschlussbänder 1 unterscheiden sich hierbei lediglich hinsichtlich der Releasekomponentenlage 21, welche mittels eines weiteren Releaseklebstoffstreifens 51 (siehe Figur 2) zusätzlich an der Kaschierung 14 des elastischen Elementes 13 lösbar angeklebt ist. Der weitere Releaseklebstoffstreifen 51 befindet sich an dem permanentbereichseitigen Ende 52 des Releasebandes 34. Insofern ist die Releasekomponentenlage 21 auch in Permanentbereich 9 des Windelverschlussbandes 1 an der Fasteningkomponentenlage 19 zusätzlich befestigbar.

Der weitere Aufbau des Windelverschlussbandes 1 aus der Figur 2 ist mit dem Aufbau des Windelverschlussbandes 1 aus der Figur 1 identisch. Um Wiederholungen hinsichtlich des Aufbaus und der Funktion der beiden Windelverschlussbänder 1 zu vermeiden, wird auf eine ausführliche Erläuterung der identischen Komponenten des Windelverschlussbandes 1 des weiteren Ausführungsbeispiels 50 verzichtet.

Es versteht sich, dass die beiden gezeigten Windelverschlussbänder 1 aus den Figuren 1 und 2 lediglich bevorzugte Ausführungsbeispiele der vorliegenden Erfindung darstellen. Insofern sind diese Windelverschlussbänder 1 nicht einschränkend auf die Erfindung zu verstehen. Insbesondere können sämtliche in den vorliegenden Unterlagen offenbarte Merkmale als erfindungswesentlich separat oder in beliebiger Kombination entsprechend ihrer Vorteile bei einem Windelverschlussband zur Anwendung kommen.

**Bezugszeichenliste:**

| | | | |
|---|---|---|---|
| 1 | Windelverschlussband | 22 | mechanischer Verschlussbereich |
| 2 | erster Halbzeugstreifennutzen | 23 | klebender Verschlussbereich |
| 3 | Halbzeugbahn | 24 | abgewandte Seite |
| 4 | Papierebene | 25 | mechanisches Verschlusselement |
| 5 | Streifen | 26 | Haken |
| 6 | Längserstreckung | 27 | Hakenträgerelement |
| 7 | Trägerband | 28 | Fasteningkleber |
| 8 | Fasteningbereich | 29 | Montagekleber |
| 9 | Permanentbereich | 30 | klebender Fasteningbereichsaufbau |
| 10 | erstes Trägerbandteil | 31 | mechanischer Fasteningbereichsaufbau |
| 11 | zweites Trägerbandteil | 32 | Targetelement |
| 12 | elastischer Bereich | 33 | Targetelementklebstoff |
| 13 | elastisches Element | 34 | Releaseband |
| 14 | Kaschierung | 35 | Releasebandklebstoff |
| 15 | erste Seite des Windelverschlussbandes | 36 | Zwischenträger |
| | | 37 | zusätzliche Releaseklebstoffschicht |
| 16 | Filmschicht | 38 | Erstreckungsbereich |
| 17 | zweite Seite des Windelverschlussbandes | 39 | Abstand |
| | | 40 | Verschlusselementhöhe |
| 18 | 3-lagiges Funktionstape | 50 | weiteres Ausführungsbeispiel |
| 19 | Fasteningkomponentenlage | 51 | weiterer Releaseklebstoffstreifen |
| 20 | Targetkomponentenlage | 52 | permanentbereichseitiges Ende |
| 21 | Releasekomponentenlage | | |

## Patentansprüche

1. Windelverschlussband (1) mit einem Permanentbereich (9) und mit einem Fasteningbereich (8), wobei der Fasteningbereich (8) einen mechanischen Verschlussbereich (22) mit mechanischen Verschlusselementen (25) umfasst, **dadurch gekennzeichnet, dass** der mechanische Verschlussbereich (22) zwischen einem permanentbereichseitig (9) des mechanischen Verschlussbereiches (22) angeordneten elastischen Bereich (12) und einem auf einer dem Permanentbereich (9) abgewandten Seite (24) des mechanischen Verschlussbereiches (22) angeordneten klebenden Verschlussbereich (23), der in dem Fasteningbereich (8) liegt, angeordnet ist und dass die mechanischen Verschlusselemente (25) des mechanischen Verschlussbereiches (22) auf wenigstens einem Trägerelement (27) angeordnet und einstückig mit dem Trägerelement (27) verbunden sind .

2. Windelverschlussband (1) nach Anspruch 1, **dadurch gekennzeichnet, dass** der Fasteningbereich (8) einen klebenden Fasteningbereichsaufbau (30) mit einer Höhe HK und einen mechanischen Fasteningbereichsaufbau (31) mit einer Höhe HM aufweist, wobei HM ≤ 2,0 x HK, vorzugsweise HM ≤ 1,7 x HK, ist.

3. Windelverschlussband (1) nach Anspruch 2, **dadurch gekennzeichnet, dass** der klebende Fasteningbereichsaufbau (30) diejenigen Komponenten des Windelverschlussbandes (1) umfasst, welche an einer einem Trägerband (7) abgewandten Seite eines Montageklebstoffes (29) zumindest teilweise im klebenden Verschlussbereich (23) angeordnet sind.

4. Windelverschlussband (1) nach Anspruch 2 oder 3, **dadurch gekennzeichnet, dass** der klebende Fasteningbereichsaufbau (30) im Wesentlichen einen Fasteningklebstoff (28), ein Targetelement (32), einen Targetelementklebstoff (33), ein Releaseband (34) und ein Releasebandklebstoff (35) umfasst.

5. Windelverschlussband (1) nach einem der vorstehenden Ansprüche, **gekennzeichnet durch** ein 3-lagiges Funktionstape (18) mit einer Fasteningkomponentenlage (19), mit einer Targetkomponentenlage (20) und mit einer Releasekomponentenlage (21).

6. Windelverschlussband (1) nach einem der Ansprüche 1 bis 3, **gekennzeichnet durch** ein 2-lagiges Funktionstape mit einer Fasteningkomponentenlage (19) und mit einer Releasekomponentenlage (20), wobei der Fasteningbereich (8) einen klebenden Fasteningbereichsaufbau (30) mit einer Höhe HK und einen mechanischen Fasteningbereichsaufbau (31) mit einer Höhe HM aufweist, wobei HM ≤ 3,5 x HK, vorzugsweise HM ≤ 3,0 x HK, ist.

7. Windelverschlussband (1) nach einem der Ansprüche 1 bis 6, **dadurch gekennzeichnet, dass** der mechanische Verschlussbereich (22) und der klebende Verschlussbereich (23) bündig nebeneinander auf dem Trägerband (7) des Windelverschlussbandes (1) angeordnet sind.

8. Windelverschlussband (1) nach einem der Ansprüche 1 bis 6, **dadurch gekennzeichnet, dass** der mechanische Verschlussbereich (22) und der klebende Verschlussbereich (23) beabstandet voneinander auf dem Trägerband (7) des Windelverschlussbandes (1) angeordnet sind.

9. Windelverschlussband (1) nach einem der vorstehenden Ansprüche, **dadurch gekennzeichnet, dass** die Verschlussbereiche (22, 23) mittels eines Montageklebstoffes (29) auf einem Trägerband (7) des Windelverschlussbandes (1) dauerhaft aufgeklebt sind.

10. Windelverschlussband (1) nach Anspruch 9, **dadurch gekennzeichnet, dass** sich der Montageklebstoff (29) auf einem Trägerband (7) des Windelverschlussbandes (1) sowohl bis unter dem mechanischen Verschlussbereich (22) als auch bis unter dem klebenden Verschlussbereich (23) erstreckt.

11. Windelverschlussband (1) nach einem der vorstehenden Ansprüche, **gekennzeichnet durch** ein Breitenverhältnis "mechanischer Verschlussbereich (22)/klebender Verschlussbereich (23)" von mindestens 1:8 und höchstens 4:1.

12. Windelverschlussband (1) nach einem der vorstehenden Ansprüche, **gekennzeichnet durch** ein Breitenverhältnis "mechanischer Verschlussbereich (22)/Targetelement (32)" von mindestens 1:9 und höchstens 2:1.

13. Windelverschlussband (1) nach einem der vorstehenden Ansprüche, **dadurch gekennzeichnet, dass** der mechanische Verschlussbereich (22) auch klebende Verschlussbereiche aufweist.

14. Windelverschlussband (1) nach einem der vorstehenden Ansprüche, **dadurch gekennzeichnet, dass** der mechanische Verschlussbereich (22) ebenfalls wenigstens teilweise elastisch ausgebildet ist.

15. Windelverschlussband (1) nach einem der vorstehenden Ansprüche, **dadurch gekennzeichnet, dass** der klebende Verschlussbereich (23) mit einem übertragbaren Targetelement (32) zusammenwirkt.

16. Windelverschlussband (1) nach einem der vorstehenden Ansprüche, **gekennzeichnet durch** ein Releaseband (34), welches das mechanische Verschlusselement (22) überdeckt.

17. Windelverschlussband (1) nach einem der vorstehenden Ansprüche, **gekennzeichnet durch** ein Releaseband (34), welches das elastische Element (13) wenigstens teilweise überdeckt.

18. Windelverschlussband (1) nach einem der vorstehenden Ansprüche, **dadurch gekennzeichnet, dass** der elastische Bereich (12) ein elastisches Element (13) umfasst, welches auf der Seite, auf welcher die Verschlusselemente (22, 23) vorgesehen sind, hervorkragt.

19. Windelverschlussband (1) nach einem der vorstehenden Ansprüche, **dadurch gekennzeichnet, dass** der elastische Bereich (12) ein elastisches Element (13) umfasst, welches mittels einer Kaschierung (14) kaschiert ist.

20. Windelverschlussband (1) nach einem der vorstehenden Ansprüche, **dadurch gekennzeichnet, dass** der elastische Bereich (12) ein elastisches Element (13) umfasst, welches an ein Trägerband (7) des Windelverschlussbandes (1) anextrudiert ist.

21. Windelverschlussband (1) nach einem der vorstehenden Ansprüche, **dadurch gekennzeichnet, dass** der elastische Bereich (12) ein elastisches Element (13) umfasst, welches einen elastisch verformbaren Kernbereich aufweist, welcher in Längserstreckung des Windelverschlussbandes (1) wenigstens 2,5 mm, vorzugsweise 3,00 mm, misst.

22. Windelverschlussband (1) nach einem der vorstehenden Ansprüche, **dadurch gekennzeichnet** durch ein Trägerband (7) mit einem fasteningbereichseitigen (8) Trägerbandteil (10) und einem permanentbereichseitigen (9) Trägerbandteil (11), wobei der fasteningbereichseitigen (8) Trägerbandteil (10) und der permanentbereichseitige (9) Trägerbandteil (11) mehr als 2,00 mm, vorzugsweise mehr als 3,00 mm, voneinander beabstandet angeordnet sind.

## Claims

1. Fastener (1) for diapers with a permanent area (9) and with a fastening area (8), wherein the fastening area (8) comprises a mechanical fastening area (22) with mechanical fastening elements (25), **characterised in that** the mechanical fastening area (22) is arranged between an elastic area (12) arranged on the permanent area side (9) of the mechanical fastening area (22) and an adhesive fastening area (23) which is in the fastening area (8) and arranged on a side (24) of the mechanical fastening area (22) facing away from the permanent area (9) and **in that** the mechanical fastening elements (25) of the mechanical fastening area (22) are arranged on at least one carrier element (27) and connected in one piece to the carrier element (27) .

2. Fastener (1) for diapers according to claim 1, **characterised in that** the fastening area (8) comprises an adhesive fastening area structure (30) with a height HK and an mechanical fastening area structures (31) with a height HM, wherein HM is ≤ 2.0 x HK, preferably
HM ≤ 1.7 x KH.

3. Fastener (1) for diapers according to claim 2 **characterised in that** the adhesive fastening area structure (30) comprises those components of the fastener (1) for diapers which are arranged on a side of an assembly adhesive (29) facing away from a carrier strip (7) at least partially in the adhesive fastening area (23).

4. Fastener (1) for diapers according to claim 2 and 3 **characterised in that** the adhesive fastening area structure (30) essentially comprises a fastening adhesive (28) a target element (32), a target element adhesive (33), a release band (34) and a release band adhesive (35).

5. Fastener (1) for diapers according to any one of the preceding claims **characterised by** a 3-layer function tape (18) with a fastening component layer (19), with a target component layer (20) and with a release component layer (21).

6. Fastener (1) for diapers according to any one of claims 1 to 3 **characterised by** a 2-layer function tape with a fastening component layer (19) and with a release component layer (20), wherein the fastening area (8) comprises an adhesive fastening area structure (30) with a height HK and a mechanical fastening area structure (31) with a height HM, wherein HM is ≤ 3.5 x HK, preferably HM ≤ 3.0 x HK.

7. Fastener (1) for diapers according to any one of claims 1 to 6 **characterised in that** the mechanical fastening area (22) and the adhesive fastening area (23) are arranged flush next to each other on the carrier band (7) of the fastener (1) for diapers.

8. Fastener (1) for diapers according to any one of claims 1 to 6 **characterised in that** the mechanical fastening area (22) and the mechanical fastening area (23) are arranged at a distance from one another on the carrier band (7) of the fastener (1) for diapers.

9. Fastener (1) for diapers according to any one of the preceding claims **characterised in that** fastening areas (22, 23) are durably adhered to a carrier band (7) of the fastener (1) for diapers by means of an assembly adhesive (29).

10. Fastener (1) for diapers according to claim 9 **characterised in that** the assembly adhesive (29) extends on a carrier band (7) of the fastener (1) for diapers both to under the mechanical fastening area (22) and to under the adhesive fastening area (23).

11. Fastener (1) for diapers according to any one of the preceding claims **characterised by** a width ratio "mechanical fastening area (22)/adhesive fastening area (23)" of at least 1:8 and at most 4:1.

12. Fastener (1) for diapers according to any one of the preceding claims **characterised by** a width ratio "mechanical fastening area (22)/target element (32)" of at least 1:9 and at most 2:1.

13. Fastener (1) for diapers according to any one of the preceding claims **characterised in that** the mechanical fastening area (22) also comprises adhesive fastening areas.

14. Fastener (1) for diapers according to any one of the preceding claims **characterised in that** the mechanical fastening area (22) is, at least in parts, also designed elastically.

15. Fastener (1) for diapers according to any one of the preceding claims, **characterised in that** the adhesive fastening area (23) interact with a transferrable target element (32).

16. Fastener (1) for diapers according to any one of the preceding claims, **characterised by** a release band (34) .

17. Fastener (1) for diapers according to any one of the preceding claims **characterised by** a release band (34) which at least partially covers the elastic element (13) .

18. Fastener (1) for diapers according to any one of the preceding claims **characterised in that** the elastic area (12) comprises an elastic element (13) which protrudes on the side on which the fastening elements (22, 23) are provided.

19. Fastener (1) for diapers according to any one of the preceding claims **characterised in that** the elastic area (12) comprises an elastic element (13) which is lined by means of a lining (14).

20. Fastener (1) for diapers according any one of the preceding claims **characterised in that** the elastic area (12) comprises an elastic element (13) which is extruded onto a carrier band (7) of the fastener (1) for diapers.

21. Fastener (1) for diapers according to any one of the preceding claims **characterised in that** the elastic area (12) comprises an elastic element (13) which has an elastically deformable core area which in the longitudinal direction of the fastener (1) for diapers measures at least 2.5 mm, preferably 3.0 mm.

22. Fastener (1) for diapers according to any one of the preceding claims **characterised by** a carrier band (7) with a carrier band section (10) on the fastening area side (8) and a carrier band section (11) on the permanent area side (9), wherein the carrier band section (10) on the fastening area side (8) and the carrier band section (11) on the permanent area side (11) are arranged at a distance from each other of more than 2.00 mm, preferably more than 3.00 mm.

## Revendications

1. Bande de fermeture d'une couche-culotte (1) dotée d'une zone permanente (9) et d'une zone de fixation (8), la zone de fixation (8) comprenant une zone de fermeture mécanique (22) avec des éléments de fermeture mécanique (25), **caractérisée en ce que** la zone de fermeture mécanique (22) est placée entre une zone élastique (12) placée du côté zone permanente (9) de la zone de fermeture mécanique (22) et une zone de fermeture adhésive (23) placée sur le côté (24) de la zone de fermeture mécanique (22) qui est opposé à la zone permanente (9), qui se situe dans la zone de fixation (8) et **en ce que** les éléments de fermeture mécanique(25) de la zone de fermeture mécanique (22) sont placés sur au moins un élément support (27) et sont assemblés en monobloc avec l'élément support (27) .

2. Bande de fermeture d'une couche-culotte (1) selon la revendication 1, **caractérisée en ce que** la zone de fixation (8) comporte une structure de zone de fixation adhésive (30) d'une hauteur HK et une structure de zone de fixation mécanique (31) d'une hauteur HM, sachant que HM ≤ 2,0 x HK, de préférence, HM ≤ 1,7 x HK.

3. Bande de fermeture d'une couche-culotte (1) selon la revendication 2, **caractérisée en ce que** la structure de zone de fixation adhésive (30) comprend les composants de la bande de fermeture d'une couche-culotte (1), lesquels sur un côté opposé à une bande support (7) d'un agent adhésif de montage (29) sont placés au moins en partie dans la zone de fermeture adhésive (23).

4. Bande de fermeture d'une couche-culotte (1) selon la revendication 2 ou 3, **caractérisée en ce que** la structure de zone de fixation adhésive (30) comprend essentiellement un agent adhésif de fixation (28), un élément cible (32), un agent adhésif d'élément cible (33), une bande de libération (34) et un agent adhésif de bande de libération (35).

5. Bande de fermeture d'une couche-culotte (1) selon l'une quelconque des revendications précédentes, **caractérisée par** un ruban fonctionnel à 3 couches (18), doté d'une couche de composant de fixation (19), d'une couche de composant cible (20) et d'une couche de composant de libération (21).

6. Bande de fermeture d'une couche-culotte (1) selon l'une quelconque des revendications 1 à 3, **caractérisée par** un ruban fonctionnel à 2 couches, doté d'une couche de composant de fixation (19) et d'une couche de composant de libération (20), la zone de fixation (8) comportant une structure de zone de fixation adhésive (30) d'une hauteur HK et une structure de zone de fixation mécanique (31) d'une hauteur HM, sachant que HM ≤ 3,5 x HK, de préférence, HM ≤ 3,0 x HK.

7. Bande de fermeture d'une couche-culotte (1) selon l'une quelconque des revendications 1 à 6, **caractérisée en ce que** la zone de fermeture mécanique (22) et la zone de fermeture adhésive (23) sont placées côte à côte, à fleur l'une de l'autre sur la bande support (7) de la bande de fermeture d'une couche-culotte (1).

8. Bande de fermeture d'une couche-culotte (1) selon l'une quelconque des revendications 1 à 6, **caractérisée en ce que** la zone de fermeture mécanique (22) et la zone de fermeture adhésive (23) sont placées avec un écart l'une par rapport à l'autre sur la bande support (7) de la bande de fermeture d'une couche-culotte (1).

9. Bande de fermeture d'une couche-culotte (1) selon l'une quelconque des revendications précédentes, **caractérisée en ce que** les zones de fermeture (22, 23) sont collées durablement, au moyen d'un agent adhésif de montage (29) sur une bande support (7) de la bande de fermeture d'une couche-culotte (1).

10. Bande de fermeture d'une couche-culotte (1) selon la revendication 9, **caractérisée en ce que** l'agent adhésif de montage (29) s'étend sur une bande support (7) de la bande de fermeture d'une couche-culotte (1), aussi bien jusqu'en dessous de la zone de fermeture mécanique (22) qu'également jusqu'en dessous de la zone de fermeture adhésive (23).

11. Bande de fermeture d'une couche-culotte (1) selon l'une quelconque des revendications précédentes, **caractérisée par** un rapport en largeur de la « zone de fermeture mécanique (22) à la zone de fermeture adhésive (23) » d'au moins 1 : 8 et d'au plus 4 : 1.

12. Bande de fermeture d'une couche-culotte (1) selon l'une quelconque des revendications précédentes, **caractérisée par** un rapport en largeur de la « zone de fermeture mécanique (22) à l'élément cible (32) » d'au moins 1 : 9 et d'au plus 2 : 1.

13. Bande de fermeture d'une couche-culotte (1) selon l'une quelconque des revendications précédentes, **caractérisée en ce que** la zone de fermeture mécanique (22) comporte aussi des zones de fermeture adhésives.

14. Bande de fermeture d'une couche-culotte (1) selon l'une quelconque des revendications précédentes, **caractérisée en ce que** la zone de fermeture mécanique (22) est également conçue au moins en partie élastique.

15. Bande de fermeture d'une couche-culotte (1) selon l'une quelconque des revendications précédentes, **caractérisée en ce que** la zone de fermeture adhésive (23) coopère avec un élément cible (32) transmissible.

16. Bande de fermeture d'une couche-culotte (1) selon l'une quelconque des revendications précédentes, **caractérisée par** une bande de libération (34), laquelle recouvre l'élément de fermeture mécanique (22) .

17. Bande de fermeture d'une couche-culotte (1) selon l'une quelconque des revendications précédentes, **caractérisée par** une bande de libération (34), laquelle recouvre au moins en partie l'élément élastique (13).

18. Bande de fermeture d'une couche-culotte (1) selon l'une quelconque des revendications précédentes, **caractérisée en ce que** la zone élastique (12) comprend un élément élastique (13) qui est en saillie sur le côté sur lequel sont prévus les éléments de fermeture (22, 23).

19. Bande de fermeture d'une couche-culotte (1) selon l'une quelconque des revendications précédentes, **caractérisée en ce que** la zone élastique (12) comprend un élément élastique (13) lequel est masqué par un pelliculage (14).

20. Bande de fermeture d'une couche-culotte (1) selon l'une quelconque des revendications précédentes, **caractérisée en ce que** la zone élastique (12) comprend un élément élastique (13) lequel est extrudé sur une bande support (7) de la bande de fermeture d'une couche-culotte (1).

21. Bande de fermeture d'une couche-culotte (1) selon l'une quelconque des revendications précédentes, **caractérisée en ce que** la zone élastique (12) comprend un élément élastique (13), lequel comporte une zone de noyau élastiquement déformable qui dans l'extension longitudinale de la bande de fermeture d'une couche-culotte (1) mesure au moins 2,5 mm, de préférence 3,00 mm.

22. Bande de fermeture d'une couche-culotte (1) selon l'une quelconque des revendications précédentes, **caractérisée par** une bande support (7) avec une partie de bande support (10) côté zone de fixation (8) et une partie de bande de support (11) côté zone permanente (9), la partie de bande support (10) côté zone de fixation (8) et la partie de bande de support (11) côté zone permanente (9) étant placées avec un écart de plus de 2,00 mm, de préférence de plus de 3,00 mm, l'une par rapport à l'autre.
